# DEMANDE DE BREVET EUROPEEN

(11) **EP 2 018 840 A1**
(43) Date de publication de la demande: **28.01.2009**
(21) Numéro de dépôt: 08290716.3
(22) Date de dépôt: 21.07.2008
(51) Int. Cl.: A61K 8/19, A61K 8/34, A61K 8/96, A61Q 19/08

(54) **Composition cosmétique et/ou dermopharmaceutique renfermant un mélange d'éléments minéraux naturels et leurs utilisations**

(30) Priorité: 23.07.2007 FR 0705322
(71) Demandeur: Ste de Production et de Conditionnement, 37310 Tauxigny (FR)
(72) Inventeur: Morvan, Jean-Roger, 37340 Hommes (FR)
(74) Mandataire: Godineau, Valérie

(57) **Abrégé**

L'invention concerne une composition cosmétique et/ou dermopharmaceutique comprenant en tant qu'actif un mélange d'éléments minéraux, issu de la roche sédimentaire d'origine marine appelée falun, renfermant du calcium en tant que constituant minéral majoritaire en pourcentage pondéral exprimé en oxyde.

Ledit mélange peut être sous la forme d'une poudre ou d'un extrait aqueux de falun en milieu neutre.

Ce mélange est notamment utilisable en tant qu'actif pour la stimulation cellulaire, la prévention du vieillissement cutané, l'action anti-rides.

## Description

La présente invention concerne le domaine des compositions cosmétiques et/ou dermopharmaceutiques comprenant du calcium en tant qu'actif ainsi que l'utilisation de ces compositions.

De nombreux sels minéraux sont nécessaires au bon équilibre de l'organisme humain. Ils représentent environ 4 % en poids du corps, le calcium étant l'élément minéral le plus abondant. En effet, il joue un rôle essentiel dans la construction et la solidité de la masse osseuse et intervient dans de nombreuses réactions biochimiques au sein des muscles, du coeur, des vaisseaux sanguins, des cellules nerveuses...

Outre l'apport journalier par les boissons et/ou les aliments, le calcium peut être apporté par voie orale au moyen de compléments sous la forme, le plus souvent, de carbonate de calcium ou de citrate de calcium. Ce calcium est, en outre, fréquemment associé à la vitamine D qui permet une meilleure assimilation par l'organisme.

Lors de recherches destinées à étudier la biodisponibilité du calcium, nous avons découvert de manière surprenante que le calcium et d'autres éléments minéraux issus de roches sédimentaires pouvaient être utilisés en tant que constituants minéraux actifs dans des compositions cosmétiques et/ou dermopharmaceutiques à application topique.

Selon la présente invention, ce mélange d'éléments minéraux est issu d'une roche sédimentaire d'origine marine, appelée falun.

Cette roche sédimentaire issue de la mer des Faluns s'étendait, il y a 15 millions d'années, sur l'Ouest de la France, en particulier dans la région de Tours actuelle. Les sédiments déposés appelés faluns ou falums sont des débris coquilliers. Cette roche sédimentaire n'a été utilisée à ce jour que pour des remblais de routes et pour l'amendement en calcaire de terres agricoles en raison de sa richesse en calcium.

Les inventeurs ont découvert de manière surprenante que l'utilisation d'une fraction micronisée de falun, directement ou après extraction à l'eau, conduisait à des propriétés intéressantes du point de vue biologique. En particulier, les essais réalisés ont montré une bioactivité bénéfique significative sur les cellules de peau humaine.

La présente invention concerne donc une composition cosmétique et/ou dermopharmaceutique comprenant en tant qu'actif un mélange d'éléments minéraux, issu de la roche sédimentaire d'origine marine appelée falun, renfermant du calcium en tant que constituant minéral majoritaire en pourcentage pondéral exprimé en oxyde.

Selon un premier mode de réalisation de l'invention, le mélange est sous la forme d'une poudre claire (de couleur ivoire) obtenue par broyage grossier et/ou micronisation du falun.

La roche sédimentaire appelée falun présente généralement des teneurs en éléments minéraux naturels suivantes, exprimées en poids d'oxydes correspondants :

| | |
|---|---|
| CaO | entre 35 et 45 % |
| SiO₂ | entre 15 et 30 % |
| Al₂O₃ | Inférieure à 5 % |
| Fe₂O₃ | Inférieure à 5 % |
| MgO | Inférieure à 5 % |
| Na₂O | Inférieure à 1 % |
| K₂O | Inférieure à 1 % |
| TiO₂ | Inférieure à 1 % |
| MnO | Inférieure à 1 % |
| P₂O₅ | Inférieure à 1 % |

et des teneurs en Ba et Sr chacune inférieure à 1 % en poids.

Les compositions cosmétiques et/ou dermopharmaceutiques peuvent renfermer cette poudre "brute" à des concentrations comprises entre 0,1 % et 100 % Elles sont notamment utilisées directement en tant que boue d'enveloppement, charge abrasive ou matière colorante (par exemple dans les compositions de fond de teint).

Selon un second mode de réalisation de l'invention, le mélange est sous la forme d'une solution aqueuse obtenue par extraction à l'eau du falun en milieu neutre, de préférence présentant un pH compris entre 5 et 9. Il s'agit donc d'un extrait naturel "enrichi" en oligoéléments, ainsi que notamment en calcium et en silice, les constituants principaux.

En effet, cette solution aqueuse peut renfermer une teneur en calcium supérieure à 200 mg de calcium par litre, ainsi que du silicium en une teneur supérieure à 20 mg de silicium par litre.

Sa teneur en carbone organique est comprise entre 5 et 15 %, et proche de 10 % en poids.

De manière avantageuse, il a été constaté que le mélange d'éléments minéraux naturels renferme du calcium et du silicium en tant que constituants principaux, selon un rapport pondéral Ca/Si compris approximativement :
- entre 1,1 et 10, de préférence entre 1,5 et 5, lorsque le mélange est sous forme d'une poudre,
- et entre 2 et 100, de préférence entre 5 et 50, lorsque le mélange est sous forme d'une solution aqueuse.

Afin de faciliter la biodisponibilité du calcium, le mélange d'éléments minéraux naturels est avantageusement associé à un stérol qui est de préférence un stérol d'origine végétale appelé phytostérol. Ce stérol d'origine végétale peut être, par exemple, le cholécalciférol ou un extrait de Persea gratissima.

La présente invention porte également sur un procédé d'obtention d'un mélange d'éléments minéraux renfermant du calcium en tant que constituant majoritaire, exprimé en poids d'oxyde, destiné à un usage cosmétique et/ou dermopharmaceutique, caractérisé en ce qu'il consiste à réduire en poudre une roche sédimentaire d'origine marine, appelée falun, afin d'obtenir une poudre de préférence micronisée, et à extraire à l'eau en milieu neutre ladite poudre, puis à procéder à une filtration et à récupérer le filtrat limpide renfermant ledit mélange d'éléments minéraux naturels solubles.

Ce procédé est particulièrement mis en oeuvre dans le second mode de réalisation de l'invention, où les compositions cosmétiques et/ou dermopharmaceutiques renferment avantageusement de 0,01 % à 50 % en poids en calcium

L'invention concerne également les utilisations dudit mélange d'éléments minéraux naturels renfermant du calcium en tant que constituant minéral majoritaire, exprimé en poids d'oxyde, ledit mélange étant issu d'une roche sédimentaire d'origine marine appelée falun en tant qu'agent actif, dans la stimulation cellulaire, l'effet anti-rides, la prévention du vieillissement cutané, dans des compositions cosmétiques et/ou dermopharmaceutiques à application topique.

Ledit mélange peut également être utilisé sous forme de solution buvable, par exemple fournie en ampoules stériles, renfermant le calcium à titre de constituant majoritaire et les oligo-éléments naturels du falun pour la préparation de médicaments destinés à la régénération tissulaire.

La présente invention est illustrée par les exemples non limitatifs suivants, en référence aux figures ci-après :
La figure 1 représente le nombre de cellules en division dans l'épiderme après 72 heures d'incubation pour trois échantillons d'Extrait Minéral Enrichi de différentes concentrations ;
La figure 2 représente le nombre de cellules en division dans l'épiderme après 48 heures d'incubation, pour différentes concentrations d'Extrait Minéral Enrichi formulé sous forme de crèmes ;
La figure 3 présente la surface occupée par des fibres élastiques sur des coupes de peau en présence ou en absence d'une solution d'élastase, pour différentes concentrations de l'Extrait Minéral Enrichi ;
La figure 4 est une courbe présentant le nombre de cellules viables après 48 heures d'incubation en fonction de la concentration en Extrait Minéral Enrichi.

### Matériau de départ :

Le matériau de départ est un falun prélevé dans la roche sédimentaire se trouvant dans la région de Tours (France).

Le falun utilisé présente l'analyse élémentaire suivante (constituants exprimés en pour cent en poids d'oxyde) :

| | |
|---|---|
| CaO | 40,98 |
| SiO₂ | 22,27 |
| Al₂O₃ | 0,50 |
| Fe₂O₃ | 0,65 |
| MgO | 0,82 |
| Na₂O | 0,19 |
| K₂O | 0,18 |
| TiO₂ | 0,02 |
| MnO | 0,02 |
| P₂O₅ | 0,09 |

ainsi que :

| | |
|---|---|
| Ba | 0,0052 |
| Sr | 0,1060 |
| Azote total | <0,05 |
| Soufre total | 0,03 |
| H₂O | 0,79 |
| H₂0 + CO₂ (1000° C) | 33,24 (soit environ 9 % de carbone organique) |

### Extraction aqueuse (des sels minéraux à partir du falun) :

La roche de falun est micronisée pour obtenir une poudre de granulométrie moyenne inférieure à 1 µm. L'extraction a été réalisée par deux méthodes différentes :

### a) Méthode statique

De l'eau déminéralisée contenant un mélange conservateur (sorbate de potassium à 0,3 % en poids, benzoate de Sodium à 0,4% en poids, déhydroacétate de sodium à 0,1% en poids et alcool benzylique à 0,6% en poids) est placée dans une cuve munie d'un agitateur et maintenue à température ambiante. La poudre de falun micronisée est introduite dans l'eau dans des proportions de 20 % en poids. L'agitation est maintenue pendant 48 heures à température ambiante (15-25° C) afin d'extraire les constituants du falun. Le mélange est ensuite filtré afin de séparer la suspension minérale résiduelle du liquide. Le filtrat limpide est récupéré et conservé dans un récipient fermé et étanche. L'extrait minéral enrichi concentré obtenu est une solution aqueuse renfermant 226 mg par litre de calcium, 16 mg par litre de silicium et notamment les oligo-éléments suivants : Al, Fe, Mg, Na, K, Sr... Le rapport calcium/silicium est ici voisin de 14.

### b) Méthode dynamique

La poudre de falun micronisée est introduite dans un sac à parois filtrantes placé dans une cuve contenant de l'eau déminéralisée et renfermant un mélange conservateur (sorbate de potassium à 0,3 % en poids, benzoate de Sodium à 0,4 % en poids, déhydroacétate de sodium à 0,1% en poids et alcool benzylique à 0,6 % en poids). Ce sac contenant 200 g de poudre de falun par litre d'eau contenu dans la cuve est maintenu au moyen d'une canne au centre de la cuve. Une pompe de circulation placée en sortie de cuve d'extraction permet de recirculer la solution d'extraction en haut de cuve pendant 48 heures à température ambiante (15-25° C) afin d'extraire les constituants du falun. La solution obtenue est conservée dans un récipient fermé et étanche avant utilisation.

L'extrait minéral enrichi obtenu sous forme d'une solution aqueuse est environ deux fois plus concentrée en éléments minéraux que celui obtenu par la méthode statique, il renferme 404 mg par litre de calcium, 40 mg par litre de silicium et notamment les oligoéléments suivants : Al, Fe, Mg, Na, K, Sr... Le rapport calcium sur silicium est ici voisin de 10.

Le produit testé dans les exemples ci-après et dénommé "extrait minéral enrichi", est constitué d'une solution aqueuse obtenue par la méthode dynamique (concentration en Ca 404 mg/L, Si 40 mg/L) renfermant en outre 7500 ppm d'un phytostérol (par exemple du cholécalciférol ou vitamine D6 ou un extrait de Persea gratissima).

### Exemple 1 : Effet sur la production de fibronectine

L'objectif de cet essai était d'évaluer l'effet de l'actif "extrait minéral enrichi" sur la synthèse de fibronectine dans un modèle de fibroblastes humains normaux adultes cultivés en monocouches.

L"'extrait minéral enrichi" a été conservé à température ambiante jusqu'à son utilisation. Il a été testé sur des monocouches confluentes de fibroblastes dermiques humains normaux obtenues en cultivant des cellules issues d'une plastie abdominale réalisée chez une femme de 47 ans.

L'activateur de référence utilisé dans cette étude est le Transforming Growth Factor β (TGF-β) à 50 ng/mL.

Les fibroblastes ont été incubés 48 heures à 37° C, sous atmosphère humide et 5 % de CO₂, en absence (milieu de culture seul) ou en présence du produit de référence, ou en présence de concentrations croissantes de l'actif à l'essai, à savoir respectivement 0,01 ; 0,1 et 1 % (v/v) d'extrait minéral enrichi. Chaque essai a été réalisé en triple exemplaire (n = 3).

A la fin de la période d'incubation, la fibronectine contenue dans les milieux de culture a été quantifiée à l'aide d'un kit E.I.A. (Enzymo Immuno Assay, commercialisé par la Société TAKARA), sensible et spécifique et les protéines contenues dans les lysats cellulaires ont été quantifiées par spectrocolorimétrie (méthode de Bradford : Bradford M. (1976) Anal. Biochem., 72, 248-254).

Les résultats sont donnés sous la forme de ng de fibronectine par µg de protéines totales du tapis cellulaire (moyennes +/- la déviation standard, S.D.) et sont présentés dans le tableau 1 ci-dessous.

**Tableau 1**

| Rapport fibronectine / Protéines totales | | | | | |
|---|---|---|---|---|---|
| | Contrôle | TGF-β 50 ng/mL | Extrait minéral enrichi (%, v/v) | | |
| | | | 0,01 | 0,1 | 1 |
| Fibronectine (ng/µg protéines) | 45,1 | 51,3 | 44,9 | 42,3 | 45,8 |
| | 46,4 | 52,8 | 43,5 | 43,3 | 44,0 |
| | 45,0 | 53,2 | 42,3 | 45,2 | 49,3 |
| Moyenne | 45,5 | 52,4 | 43,5 | 43,6 | 46,4 |
| S.D. | 0,8 | 1,0 | 1,3 | 1,5 | 2,7 |
| % Contrôle | 100,0 | 115,3 | 95,7 | 95,8 | 101,9 |

| | | | | | |
|---|---|---|---|---|---|
| S.D. : déviation standard | | | | | |

La significativité statistique des différences observées entre les conditions "contrôle" et "produit de référence", a été évaluée par un test de Student (Student t-test ; p<0,001). La significativité statistique des différences observées entre les conditions "contrôle" et "produit à l'essai", a été évaluée par une analyse de la variance à un facteur (One Way ANOVA).

Le TGF-β à 50 ng/mL, utilisé comme produit de référence, augmente significativement la production de fibronectine des cellules en culture de 15,3 % (p<0,001).

Les résultats présentés dans le tableau 1 montrent que dans les conditions expérimentales retenues, l'actif "Extrait minéral enrichi" n'est pas capable d'augmenter la production de fibronectine de fibroblastes dermiques humains normaux adultes.

### Exemple 2 : Effet sur la néosynthèse de procollagène de type I

L'objectif de cet essai était d'évaluer l'effet de l'actif "Extrait minéral enrichi" sur la synthèse de procollagène de type I sans un modèle de fibroblastes humains normaux adultes cultivés en monocouches. L"'extrait minéral enrichi" a été conservé à température ambiante jusqu'à son utilisation.

Il a été testé sur des monocouches confluentes de fibroblastes dermiques humains normaux obtenues en cultivant des cellules issues d'une plastie abdominale réalisée chez une femme de 47 ans.

L'activateur de référence utilisé dans cette étude est le Transforming Growth Factor β (TGF-β) à 50 ng/mL.

Les fibroblastes ont été incubés 48 heures à 37° C, sous atmosphère humide et 5 % de CO₂, en absence (milieu de culture seul) ou en présence du produit de référence, ou en présence de concentrations croissantes de l'actif à l'essai, à savoir respectivement 0,01 ; 0,1 et 1 % (v/v) d'extrait minéral enrichi. Chaque essai a été réalisé en trois exemplaires (n=3).

A la fin de la période d'incubation, le procollagène de type I contenu dans les lysats cellulaires et dans les milieux de culture (dosage du procollagène I "total") a été quantifié à l'aide d'un kit E.L.I.S.A. sensible et spécifique, et les protéines contenues dans les lysats cellulaires ont été quantifiées par spectrocolorimétrie (méthode de Bradford : Bradford M. (1976) Anal. Biochem., 72, 248-254).

Les résultats, donnés sous la forme de ng de procollagène de type I C-Peptide par µg de protéines totales du tapis cellulaire (moyennes +/- la déviation standard, S.D.) sont présentés dans le tableau 2.

**Tableau 2**

| Rapport procollagène de type I / Protéines totales | | | | | |
|---|---|---|---|---|---|
| | Témoin | TGF-β 10 ng/mL | Extrait minéral enrichi (%, v/v) | | |
| | | | 0,01 | 0,1 | 1 |
| Procollagène I total (ng/µg protéines) | 50,50 | 90,74 | 72,40 | 76,62 | 58,18 |
| | 65,29 | 98,34 | 83,43 | 72,53 | 72,31 |
| | 69,78 | 97,86 | 65,70 | 91,63 | 63,03 |
| Moyenne | 61,86 | 95,64** | 73,84 | 80,26+ | 64,51 |
| S.D. | 10,08 | 4,26 | 8,95 | 10,06 | 7,18 |
| % Témoin | 100,0 | 154,6 | 119,4 | 129,8 | 104,3 |

| | | | | | |
|---|---|---|---|---|---|
| S.D. : déviation standard ** : moyenne significativement différente de celle du groupe "témoin" (p<0,01) + : moyenne significativement différente de celle du groupe "témoin" (p<0,09) | | | | | |

La significativité statistique des différences observées entre le "témoin" et le "produit de référence" a été évaluée par un test de Student *(Student t-test* ; ** : p<0,05).

La significativité statistique des différences observées entre le "témoin" et l'''extrait minéral enrichi à 0,1 %", a été évaluée par un test de Student *(Student t-test* ; + : p<0,09).

Les résultats présentés dans le tableau 2 montrent que dans les conditions expérimentales retenues, le TGF-β à 50 ng/ml, utilisé comme produit de référence, augmente la production fibroblastique du procollagène de type I de 54,6 % (p<0,01), et que l'actif "extrait minéral enrichi" à 0,1 % est capable également d'augmenter notablement la production de procollagène de type I de fibroblastes dermiques humains normaux adultes. Cette augmentation est significative (+ 29,8 %) pour l'extrait minéral enrichi dosé à 0,1 % (v/v).

Cet actif améliore la fermeté cutanée. Il peut donc être incorporé dans des compositions à activité anti-âge et anti-rides.

### Exemple 3 : Effet sur le nombre de cellules en division dans l'épiderme

L'objectif de cet essai est d'évaluer l'effet de l'actif "Extrait minéral enrichi" sur la réplication des cellules des couches basales de l'épiderme d'explants de peau humaine normale maintenus en survie.

L"'extrait minéral enrichi", conservé à température ambiante jusqu'à son utilisation, a été testé sur des explants de peau humaine normale préparés à partir d'un prélèvement de peau (déchet opératoire) réalisé chez une femme âgée de 44 ans. Ces explants ont été maintenus en survie dans du milieu de culture pendant toute la durée de l'expérience, soit 72 heures.

Le produit de référence utilisé dans cette étude est le sérum de veau foetal (SVF) à 10 % (v/v).

Les explants ont été incubés pendant 72 heures en absence (milieu de culture seul = témoin) ou en présence du produit de référence (dilué dans le milieu de culture) ou en présence de l'actif à l'essai (dilué dans le milieu de culture). Les milieux de culture, contenant ou non le produit de référence et l'actif à l'essai, ont été renouvelés toutes les 24 heures.

Tous les essais ont été réalisés en trois exemplaires (3 explants par concentration d'EME).

A la fin de la période d'incubation, les explants ont été fixés dans du paraformaldéhyde à 10 %. Après inclusion dans des blocs de paraffine, des coupes d'explant ont été réalisées à l'aide d'un microtome.

La présence de cellules en division dans les couches basales de l'épiderme a été ensuite révélée par un marquage immunohistochimique de la protéine Ki67 (protéine exprimée dans le noyau des cellules en division (Marzullo F et al, Pathologica, 80, 279-85 - 1988)).

Les cellules positives ont été comptées sur chaque coupe (analyse de 10 champs microscopiques sélectionnés au hasard, par coupe). Les résultats sont donnés sous la forme du nombre de cellules "positives" (exprimant la protéine Ki67) par champ microscopique analysé (moyennes +/- la déviation standard, S.D.), et sont la significativité statistique des différences observées entre les conditions "Contrôle" et "produit de référence", a été évaluée par un test de Mann Whitney *(Mann Whitney Rank Sum Test* ; *** : p<0,001).

La significativité statistique des différences observées entre les conditions "Contrôle" et "Produit à l'essai", a été évaluée par une analyse de la variance à un facteur sur les rangs (One Way ANOVA on ranks), suivie d'un test de Dunnett (* : p<0,05).

Dans les conditions expérimentales retenues, le SVF à 10 % utilisé comme produit de référence, augmente significativement (multiplie par 7, p<0,01) le nombre de cellules en division dans les couches basales de l'épiderme après 72 heures d'incubation.

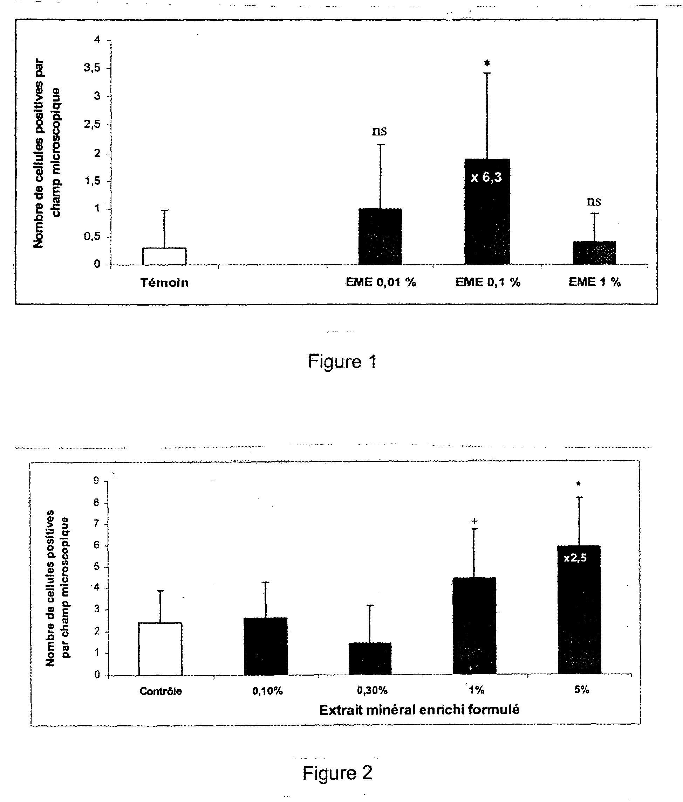

Les résultats présentés sur la figure 1 montrent que dans des conditions expérimentales identiques, le nombre de cellules en division, présentes dans les couches basales de l'épiderme après 72 heures d'incubation, est également significativement supérieur en présence de l'actif "Extrait Minéral Enrichi" (EME), à savoir :
multiplié par 3,3 (ns) pour l'EME à 0,01 %
multiplié par 6,3 (p<0,05) pour l'EME à 0,1 %
et multiplié par 1,3 (ns) pour l'EME à 1 %.

On constate dans cet exemple que l'extrait minéral enrichi à 0,1 % est significativement plus efficace qu'aux autres concentrations, supérieures ou inférieures.

Cet actif améliore la division cellulaire. Il peut donc être incorporé dans des compositions à activité anti-âge.

### Exemple 4 :

L'exemple 3 a été reproduit, en utilisant comme produit de départ des crèmes dont la formulation générale est la suivante (% en poids) :

| Nom INCI | % |
|---|---|
| Aqua | 74,9 à 70 |
| Algin | 0,5 |
| Coco Nucifera Oil | 10 |
| Glyceryl Stearate | 8 |
| Persea Gratissima Oil Unsaponifiables (sterol) | 0,2 |
| Cetearyl Glucoside | 2,5 |
| Cetearyl Alcohol | 2,5 |
| Sodium Dehydroacetate | 0,3 |
| Benzyl Alcohol | 0,3 |
| Tocopheryl Acetate | 0,2 |
| Extrait minéral *(1)* | 0,1 à 5 |
| Sodium Benzoate | 0,3 |
| Potassium Sorbate | 0,2 |

| | |
|---|---|
| *(1)* l'extrait minéral enrichi a été obtenu par extraction aqueuse statique, sa concentration en calcium est de 226 mg/L) | |

Les crèmes ont été conservées à température ambiante jusqu'à leur utilisation.

Les explants ont été incubés pendant 48 heures en absence (milieu de culture seul = contrôle) ou en présence des actifs à l'essai (appliqués en topique à la surface des explants, à raison de 10 µl par explant). Les milieux de culture, contenant ou non le produit de référence et l'actif à l'essai ont été renouvelés toutes les 24 heures.

Les résultats présentés dans la figure 2 montrent que dans les conditions expérimentales retenues, le nombre de cellules en division présentes dans les couches basales de l'épiderme après 48 heures d'incubation, est significativement supérieur en présence de la crème renfermant l'actif "Extrait Minéral Enrichi" :
Formulation à 1 % multipliée par 1,83 (ns)
Formulation à 5 % multipliée par 2,46 (p<0,05)

La formulation à 5 % présente donc un effet positif sur la prolifération (et donc le renouvellement) des cellules des couches basales de l'épiderme humain.

### Exemple 5 : Activité anti-élastasique

L'objectif de cet essai était d'évaluer l'activité anti-élastasique du produit "extrait minéral enrichi", dans un modèle de coupes de peau humaine.

L"'extrait minéral enrichi" a été conservé à température ambiante jusqu'à son utilisation.

Des cryocoupes de peau humaine ont été utilisées comme substrat de la réaction. La peau utilisée provenait d'une plastie réalisée chez un sujet féminin de 55 ans.

L'inhibiteur de référence utilisé dans cet essai était la 1,10 phénanthroline à 10⁻² M.

Les cryocoupes de peau ont été pré-incubées pendant 30 minutes à 37° C en absence (tampon seul) ou en présence du produit de référence, ou en présence de l'actif à l'essai, respectivement à des teneurs : 0,05 ; 0,1 et 0,5 % (v/v), l'actif étant solubilisé directement dans le tampon d'essai.

Les cryocoupes ont ensuite été incubées pendant 3 heures à 37° C dans les mêmes conditions (c'est-à-dire en absence ou en présence du produit de référence ou des actifs à l'essai), en absence (témoins sans enzyme) ou en présence d'une solution d'élastase à 0,5 U/mL.

A la fin de la période d'incubation, les coupes ont été rincées avec du tampon Tris puis colorées à l'orcéine, en vue du marquage des fibres élastiques.

Chaque condition expérimentale "actif + enzyme" a été réalisée en double exemplaire (n = 2).

Pour chaque condition expérimentale retenue, l'activité de l'élastase a été mesurée sur les coupes de peau colorées à l'orcéine, par quantification de la surface occupée par les fibres élastiques. Elle a été réalisée par analyse d'images sur dix régions différentes de 1300 x 1030 pixels chacune, pour une surface totale analysée supérieure à 360.000 µm² (1 pixel = 0,027 µm²) par condition expérimentale.

Les résultats sont donnés sous forme de pourcentages d'inhibition de la digestion des fibres élastiques (moyennes +/- la déviation standard, S.D.).

La significativité statistique des différences observées entre les conditions "témoin" et "Elastase" a été évaluée à l'aide d'un test de Mann Whitney (Mann Whitney Rank Sum test, p<0,001).

La significativité statistique des différences observées entre les conditions "Elastase" et "Elastase + Phénanthroline" a été évaluée à l'aide d'un test de Mann Whitney (Mann Whitney Rank Sum test, p<0,001).

La significativité statistique des différences observées entre les conditions "Elastase" d'une part, et "Actifs + Elastase" d'autre part, a été évaluée par une analyse de la variance à un facteur (one way ANOVA) (p<0,05).

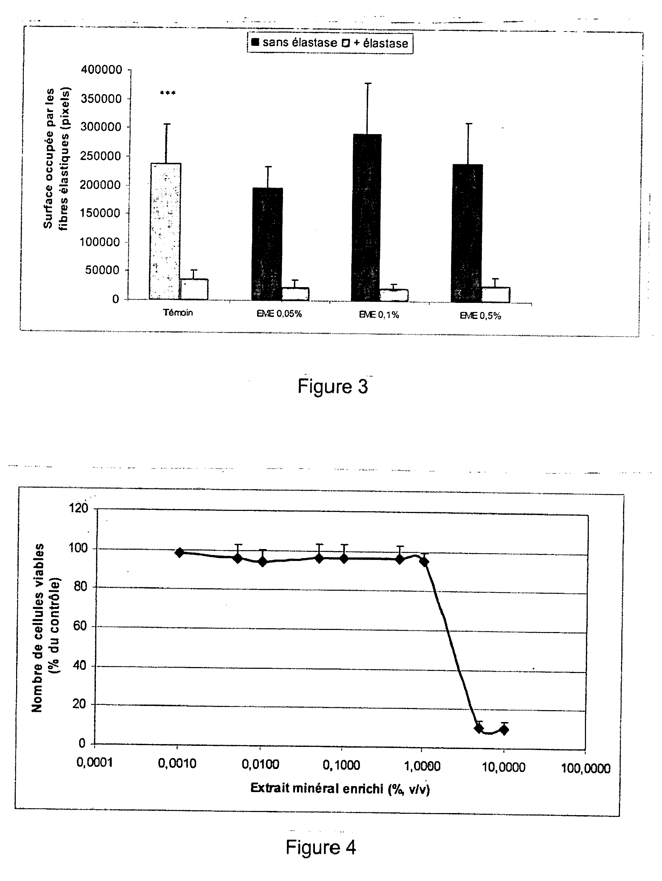

Les résultats présentés dans la figure 3 montrent que dans les conditions expérimentales retenues, l'actif "extrait minéral enrichi" ne présente pas d'activité anti-élastasique significative, au contraire de la 1,10 phénanthroline à 10⁻² M, utilisée comme inhibiteur de référence qui inhibe significativement l'activité de l'enzyme étudiée de 47,1 % (p<0,001, t-test).

### Exemple 6 : Cytotoxicité

L'objectif de cet essai était d'évaluer la cytotoxicité de l'actif "extrait minéral enrichi" vis-à-vis de fibroblastes dermiques humains normaux cultivés en monocouches.

Le produit "extrait minéral enrichi" a été conservé à température ambiante jusqu'à son utilisation et testé sur des fibroblastes humains normaux obtenus à partir d'une plastie abdominale réalisée chez une femme de 33 ans.

Pour la réalisation des essais, ces cellules ont été cultivées jusqu'à l'obtention de monocouches confluentes.

Les cellules ont été incubées pendant 48 heures en absence (contrôle) ou en présence de concentrations croissantes (0,001 ; 0,005 ; 0,01 ; 0,05 ; 0,1 ; 0,5 ; 1 ; 5 et 10 %, v/v) de l'actif "extrait minéral enrichi" dans le milieu d'incubation des cellules.

A la fin de la période d'incubation, la viabilité des cellules a été évaluée par une méthode spectrophotométrique de dosage de l'activité des phosphatases intracellulaires.

Le principe du dosage, décrit par Yang T. et al. (Anal. Biochem., 24, 103-108 (1996)), le p-nitrophényl phosphate est transformé en p-nitrophénol par les phosphatases intracellulaires des cellules viables. L'absorbance du p-nitrophénol à 405 nm est directement proportionnelle au nombre des cellules viables présentes dans les puits de culture.

Les résultats sont donnés sous la forme de pourcentages de cellules viables (moyennes +/- la déviation standard, S.D.) et sont présentés sur la figure 4.

Les résultats présentés dans la figure 4 montrent que dans les conditions expérimentales retenues, après 48 heures d'incubation, l'actif "extrait minéral enrichi" diminue significativement le nombre de fibroblastes viables présents dans les puits de culture. Cet effet est détectable dès la dose de 5 % (v/v) et maximal pour une concentration de 10 % (v/v).

Dans les conditions expérimentales retenues, l'actif "extrait minéral enrichi" ne présente donc un effet cytotoxique vis-à-vis de fibroblastes dermiques humains normaux cultivés en monocouches, qu'à partir de la dose 5 % (v/v).

En conséquence, il ne présente pas d'effet cytotoxique pour les concentrations intéressantes relevées dans les exemples 1 à 5, à savoir entre 0,001 et 1 % (v/v).

## Revendications

1. Composition cosmétique et/ou dermopharmaceutique comprenant en tant qu'actif un mélange d'éléments minéraux, issu de la roche sédimentaire d'origine marine appelée falun, renfermant du calcium en tant que constituant minéral majoritaire en pourcentage pondéral exprimé en oxyde.

2. Composition selon la revendication 1,
**caractérisée en ce que** le mélange est sous la forme d'une poudre obtenue par broyage grossier et/ou micronisation du falun.

3. Composition selon la revendication 1 ou 2,
**caractérisée en ce que** la roche sédimentaire appelée falun présente des teneurs en éléments minéraux naturels suivantes, exprimées en poids d'oxydes correspondants :
| | |
|---|---|
| CaO | entre 35 et 45% |
| SiO₂ | entre 15 et 30% |
| Al₂O₃ | Inférieure à 5% |
| Fe₂O₃ | Inférieure à 5% |
| MgO | Inférieure à 5% |
| Na₂O | Inférieure à 1% |
| K₂O | Inférieure à 1% |
| TiO₂ | Inférieure à 1% |
| MnO | Inférieure à 1% |
| P₂O₅ | Inférieure à 1% |
et des teneurs en Ba et Sr chacune inférieure à 1 % en poids.

4. Composition selon l'une quelconque des revendications 1 à 3,
**caractérisée en ce que** le mélange est sous la forme d'une solution aqueuse obtenue par extraction à l'eau du falun en milieu neutre, de préférence présentant un pH compris entre 5 et 9.

5. Composition selon la revendication 4,
**caractérisée en ce que** la solution aqueuse renferme une teneur en calcium supérieure à 200 mg Ca/L.

6. Composition selon l'une quelconque des revendications précédentes,
**caractérisée en ce que** la solution aqueuse renferme du silicium en une teneur supérieure à 20 mg Si/L.

7. Composition selon l'une quelconque des revendications précédentes,
**caractérisée en ce que** le mélange renferme du calcium et du silicium en tant que constituants principaux, selon un rapport pondéral Ca/Si compris approximativement :
- entre 1,1 et 10, de préférence entre 1,5 et 5, lorsque le mélange est sous forme d'une poudre,
- et entre 2 et 100, de préférence entre 5 et 50, lorsque le mélange est sous forme d'une solution aqueuse.

8. Composition selon l'une quelconque des revendications précédentes,
**caractérisée en ce que** le mélange d'éléments minéraux est associé à un stérol, de préférence un stérol d'origine végétale.

9. Composition selon la revendication 7,
**caractérisée en ce que** le stérol d'origine végétale est le cholécalciférol ou un extrait de Persea gratissima.

10. Procédé d'obtention d'un mélange d'éléments minéraux renfermant du calcium en tant que constituant majoritaire, exprimé en poids d'oxyde, destiné à un usage cosmétique et/ou dermopharmaceutique,
**caractérisé en ce qu'**il consiste à réduire en poudre une roche sédimentaire d'origine marine, appelée falun, afin d'obtenir une poudre de préférence micronisée, et à extraire à l'eau en milieu neutre ladite poudre, puis à procéder à une filtration et à récupérer le filtrat limpide renfermant ledit mélange d'éléments minéraux naturels solubles.

11. Utilisation en tant qu'agent actif pour la stimulation cellulaire dans une composition cosmétique et/ou dermopharmaceutique à application topique, d'un mélange d'éléments minéraux naturels, renfermant du calcium en tant que constituant minéral majoritaire, exprimé en poids d'oxyde, ledit mélange étant issu d'une roche sédimentaire d'origine marine appelée falun.

12. Utilisation en tant qu'agent actif à effet anti-rides ou pour la prévention du vieillissement cutané dans une composition cosmétique et/ou dermopharmaceutique à application topique, d'un mélange d'éléments minéraux naturels, renfermant du calcium en tant que constituant minéral majoritaire, exprimé en poids d'oxyde, ledit mélange étant issu d'une roche sédimentaire d'origine marine appelée falun.

13. Utilisation pour la préparation d'un médicament destiné à la régénération tissulaire d'un mélange d'éléments minéraux naturels, renfermant du calcium en tant que constituant minéral majoritaire, exprimé en poids d'oxyde, ledit mélange étant issu d'une roche sédimentaire d'origine marine appelée falun.

14. Utilisation selon l'une quelconque des revendications 11 à 13, **caractérisée en ce que** le mélange d'éléments minéraux naturels est associé à au moins un stérol d'origine végétale.
